# EUROPEAN PATENT SPECIFICATION

(11) **EP 2 196 223 B1**
(45) Date of publication and mention of the grant of the patent: **03.09.2014**
(21) Application number: 08170839.8
(22) Date of filing: 05.12.2008
(51) Int. Cl.: A61L 2/26, A61L 2/07

(54) **Sterilization chamber made of polyethersulfone, process for its manufacture and sterilization apparatus comprising this chamber**
Sterilisationskammer aus Polyethersulfon, Verfahren zu deren Herstellung und Sterilisationsgerät, das diese Kammer umfasst
Chambre de stérilisation fabriquée à partir de polyéthersulfone, son procédé de fabrication et appareil de stérilisation comportant cette chambre

(43) Date of publication of application: 16.06.2010
(73) Proprietor: Solvay Specialty Polymers USA, LLC., Alpharetta, GA 30005 (US)
(72) Inventor: Bonnadier, Jean-Baptiste, 1180, Brussels (BE)
(74) Representative: Vande Gucht, Anne

(56) References cited:
- WO-A-02/100446
- DE-A1-102007 002 105
- JP-A- 2002 308 229
- US-A- 4 671 943
- US-A- 5 326 834
- US-A1- 2004 036 039
- US-A1- 2006 045 798
- US-A1- 2007 145 304

## Description

The present invention relates to a sterilization chamber made of a polymeric material, a process for its manufacture and a sterilization apparatus comprising this sterilization chamber.

Sterilization apparatuses are commonly used in health care and medicine. Sterilization apparatuses as they are commonly used in small variants in medical and dental clinics, and in large variants in pharmaceutical plants may largely vary in size, but usually have in common that they comprise a sterilization chamber in which goods to be sterilized are placed. In the sterilization chamber there may be several inlets/outlets for steam, water or the like, depending on the kind of sterilization that is to be performed in it. Furthermore, means for pressurizing and heating the interior space of said chamber are provided.

Sterilization chambers are quite often made out of stainless metal material. Stainless metal material one has been so far *"the"* material of choice for this application, because it exhibits high strength, high toughness and high fatigue resistance. High toughness and high fatigue resistance are obviously key properties for sterilization chambers, because these ones see pressures over 2 bars, and also steam.

However, there are some drawbacks associated with sterilization chambers made of stainless metal material. For example, their manufacture may be somewhat time-consuming, since it requires welding or the like. Furthermore, a lot of energy is lost when heating such chambers, since some of the added energy is used for heating the chamber material, resulting in a somewhat ineffective sterilization process.

It has already been attempted to overcome these drawbacks, by proposing sterilization chambers made of certain polymeric materials, in vain.

For example, WO 02/100446 A (to GETINGE) relates to a sterilization chamber for use in a sterilization device or the like. The chamber is adapted to enclose goods to be sterilized during a sterilization process and has a self supported structure being essentially manufactured from a polymeric material. As polymeric material, WO 02/100446 A discloses the use of a polyamide material or of a composite material, wherein the composite material preferably comprises a carbon fiber rowing weave and a concatenating polymer material.

WO 02/10047 A (also to GETINGE) discloses a disinfection chamber for use in a disinfection device for health care goods, such as bed pans, urine bottles and the like, the chamber in use being intended to receive the goods which are to be disinfected. The chamber has a self supported structure which is substantially made of a polymer material. The polymeric material is preferably a propylene plastic material.

Finally, WO 04/20001 (still to GETINGE) discloses a sterilization apparatus comprising a sterilization unit and a processing unit which are mechanically separated from each other, wherein the sterilization unit comprises a sterilization chamber may be manufactured from a polymeric material.

The prior art plastic-made sterilization chambers (wherein the plastic material may be a polyamide, a composite or a polypropylene, as above detailed) do not exhibit the high toughness and the high fatigue resistance as desired for the application. As a result thereof, they have been essentially unable so far to replace efficiently stainless steel metal-based sterilization chambers.

On the other hand, poly(aryl ether sulfone)s have already been proposed for various sterile applications in the medical field, including membrane filters, transfer bags, autoclavable containers and trays, and lids thereof. For example, DE 197 28 217 A discloses the use of for example polysulfone membrane filters for the separation of gaseous components in the water supply of sterilizators. US 2004/0081601 A1 discloses an integrity testable autoclavable sterile transfer bag having section formed from a plastic, possibly a polyethersulfone or polyphenylsulfone. US 5,326,834 describes an autoclavable container or storage tray made out of certain poly(aryl ether sulfone)s, which exhibit excellent environmental stress-cracking resistance when exposed to various chemicals. US 2007/0212277 A1 relates to a medical instrument container system comprising trays molded entirely of a rigid plastic able to withstand cleaning and sterilization processes, e.g. polyphenylsulfone. US 2004/02566268 A1 describes a sterile container, in particular for the holding and sterile storage of surgical instruments or material, which comprises a lid (for closing the holding space) which may be made out of a plastic material such as polyphenylene sulfone. US 4,671,943 relates to containers for sterilizing, and storing and presenting surgical instruments and materials in a sterile condition. US 4,671,943 further describes that transparent heat resistant plastics including polycarbonates preferably polysulfones, polyethersulfones and polyetherimides may be used to form the container base member, tray and their corresponding covers or merely the covers of said containers.

None of the sterile medical applications of the prior art using poly(aryl ether sulfone)s have requirements similar to the sterilization chambers of present concern. For example, medical trays, which are used for cleaning of medical equipment, have actual contact with cleaning chemicals ; that's why environmental stress cracking resistance is critical for this first application. Medical trays do no see pressure. Sterilization chambers, on the other hand, see pressures over 2 bars, in addition to steam ; that's why fatigue and toughness properties are so important for this other application.

There is a need for sterilization chambers that would present the advantages of the known plastic-made sterilization chambers, while exhibiting a high toughness and high fatigue resistance, as can be achieved by the known stainless steel metal-made sterilization chambers.

These sterilization chambers would thus desirably be easy and quick to manufacture, since they would require no welding. Furthermore, the energy losses when heating the chambers would be low, since the heating energy for heating the chamber materials would be low. In addition, the goods to be sterilized would be easily heated since the heating of the chamber material would not consume much energy. Thus, fast and energy efficient sterilization cycles could be realized. The sterilization chambers would also desirably exhibit a high toughness and a high fatigue resistance, and provide long-term stability under high temperature and hydrolytic conditions as are encountered during the typical use of sterilization chambers.

An object of the present invention is to provide a sterilization chamber which overcomes the problems of the prior art. In particular, it is an object of the present invention to provide a sterilization chamber which meets the above addressed needs.

The present invention thus relates to a sterilization chamber as defined in claim 1.

In general, above 50 wt. % of the sterilization chamber, based on its total weight, is composed of the polymeric material. Preferably, above 75 wt. % of the sterilization chamber is composed of the polymeric material. More preferably, above 90 wt. % of the sterilization chamber is composed of the polymeric material. Still more preferably, the sterilization chamber consists essentially of the polymeric material. The most preferably, the sterilization chamber consists of the polymeric material.

The polymeric material may comprise the poly(aryl ether sulfone) (P) in a weight amount of at least 10 %, at least 30 % or at least 50 %, based on the total weight of the polymeric material. Preferably, the polymeric material comprises the poly(aryl ether sulfone) (P) in a weight amount of at least 70 %, based on the total weight of the polymeric material. More preferably, the polymeric material comprises the poly(aryl ether sulfone) (P) in a weight amount of at least 90 %, if not at least 95 %, based on the total weight of the polymeric material. Still more preferably, the polymeric material consists essentially of the poly(aryl ether sulfone) (P). The most preferably, it consists essentially of the poly(aryl ether sulfone) (P).

The poly(aryl ether sulfone) (P) has a advantageously a weight average molecular weight in the range of from 20,000 to 100,000. Preferably, the poly(aryl ether sulfone) (P) has a weight average molecular weight in the range of from 40,000 to 70,000. The weight average molecular weight can be determined by Gel Permeation Chromatography using conventional polystyrene calibration standards.

As detailed hereinafter, excellent results are obtained when essentially all (preferably, all) the recurring units of the poly(aryl ether sulfone) (P) are of formula (D) :

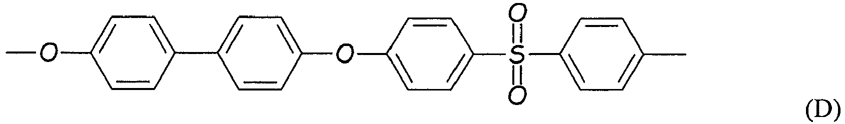

In the present invention, the poly(aryl ether sulfone) (P) is a poly(biphenyl ether sulfone) (P1).

For the purpose of the present invention,
a poly(biphenyl ether sulfone) (P1) is intended to denote any polymer of which more than 50 wt. % of the recurring units are recurring units (R1) of one or more formulae containing at least one p-phenylene group : 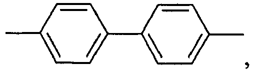 at least one ether group (-O-) and at least one sulfone group [-S(=O)₂-].

Recurring units (R1) are preferably of one ore more formulae of the general type : 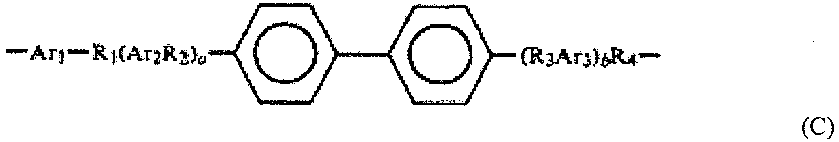 wherein R₁ through R₄ are -O-, -SO₂-, -S-, -CO-,
with the proviso that at least one of R₁ through R₄ is -SO₂- and at least one of R₁ through R₄ is -O- ; Ar₁, Ar₂ and Ar₃ are arylene groups containing 6 to 24 carbon atoms, and are preferably phenylene or p-biphenylene ; and a and b are either 0 or 1.

More preferably, recurring units (R1) are chosen from 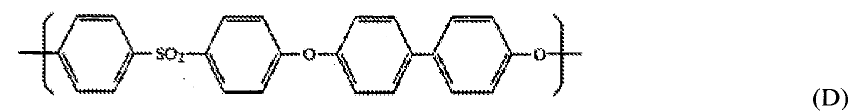 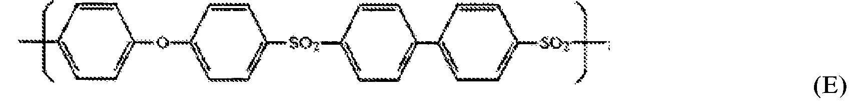 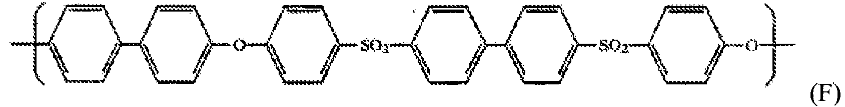 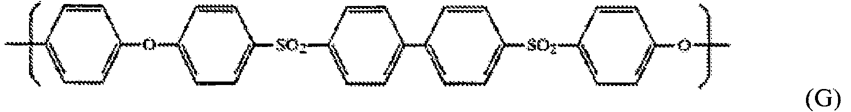 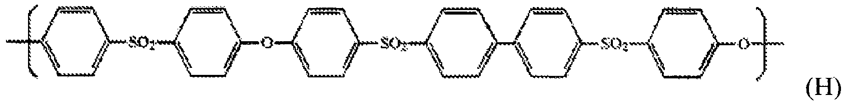 and mixtures thereof.

Still more preferably, recurring units (R1) are chosen from 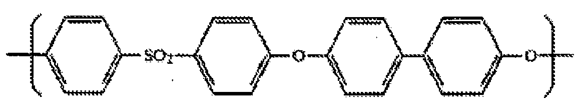 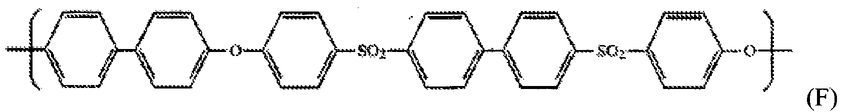 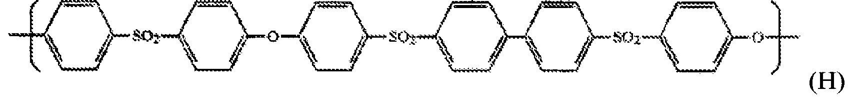 and mixtures thereof.

The most preferably, recurring units (R1) are

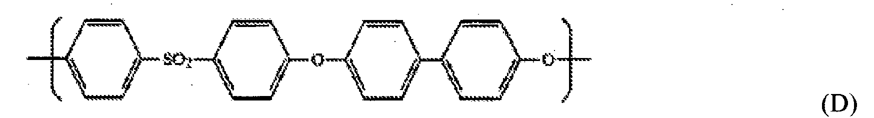

For the purpose of the present invention, a polyphenylsulfone is intended to denote any polymer of which more than 50 wt. % of the recurring units are recurring units (R1) of formula (D), while a polyphenylsulfone homopolymer is intended to denote any polymer of which essentially all (and, preferably, all) the recurring units are recurring units (R1) of formula (D).

The poly(biphenyl ether sulfone) (P1) may be notably a homopolymer or a copolymer such as a random or block copolymer. When the poly(biphenyl ether sulfone) (P1) is a copolymer, its recurring units may notably be composed essentially of (or composed of) (i) recurring units (R1) of at least two different formulae chosen from formulae (D) to (H), or (ii) recurring units (R1) of one or more formulae (D) to (H) and recurring units (R1*), different from recurring units (R1), such as : 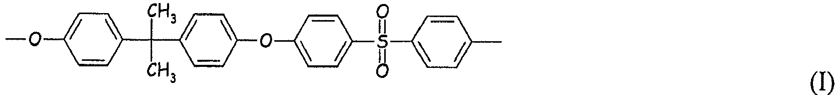 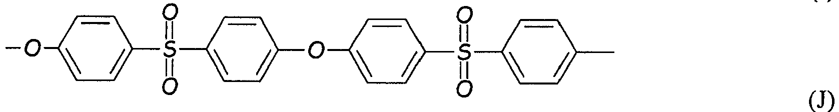 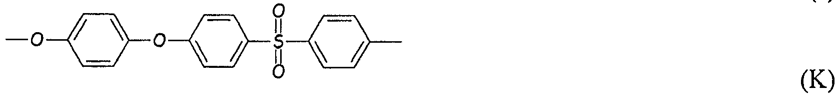 and mixtures thereof.

Preferably more than 90 wt. %, and more preferably more than 95 wt. % of the recurring units of the poly(biphenyl ether sulfone) (P1) are recurring units (R1). Still more preferably, essentially all the recurring units of the poly(biphenyl ether sulfone) are recurring units (R1). The most preferably, all the recurring units of the poly(biphenyl ether sulfone) are recurring units (R1).

Excellent results are obtained when the poly(biphenyl ether sulfone) (P1) is a polyphenylsulfone homopolymer, i.e. a polymer of which essentially (and, preferably, all) the recurring units are of formula (H). RADEL^{®} R polyphenylsulfone from SOLVAY ADVANCED POLYMERS, L.L.C. is an example of a polyphenylsulfone homopolymer.

The poly(biphenyl ether sulfone) (P1) can be prepared by any method. Methods well known in the art are those described in U.S. Pat. Nos. 3,634,355 ; 4,008,203 ; 4,108,837 and 4,175,175, the whole contents of which is herein incorporated by reference.

The poly(aryl ether sulfone) (P) is a poly(biphenyl ether sulfone) (P1). More preferably, the poly(aryl ether sulfone) (P) is a polyphenylsulfone. Still more preferably, the poly(aryl ether sulfone) (P) is a polyphenylsulfone homopolymer.

The polymeric material may contain one and only one poly(aryl ether sulfone) (P). Alternatively, the polymeric material may contain two or more poly(aryl ether sulfone)s (P) ; for example, it may contain two or more poly(biphenyl ether sulfone)s (P1) (in particular, two or more polyphenylsulfone homopolymers), or it may contain at least one poly(biphenyl ether sulfone) (P1) and at least one polyethersulfone (P3).

The poly(aryl ether sulfone) (P) which is used according to the present invention may be prepared by various methods, for example by the so-called carbonate method. Generally described, the process is conducted by contacting substantially equimolar amounts of at least one aromatic bishydroxy monomer, e.g. 4-4' bisphenol A, 4-4' bisphenol S, or 4,4'-biphenol and at least one dihalodiarylsulfone, e.g., 4,4'-dichlorodiphenyl sulfone, 4,4'-difluorodiphenyl sulfone or the like, with from about 0.5 to about 1.1 mole, preferably from about 1.01 to about 1.1 mole, more preferably from about 1.05 to about 1.1 mole of an alkali metal carbonate, preferably potassium carbonate, per mole of hydroxyl group.

The components are generally dissolved or dispersed in a solvent mixture comprising a polar aprotic solvent together with a solvent which forms an azeotrope with water, whereby water formed as a byproduct during the polymerization may be removed by azeotropic distillation continuously throughout the polymerization.

The polar aprotic solvents employed are those generally known in the art and widely used for the manufacture of poly(aryl ether sulfone)s. For example, the sulfur-containing solvents known and generically described in the art as dialkyl sulfoxides and dialkylsulfones wherein the alkyl groups may contain from 1 to 8 carbon atoms, including cyclic alkylidene analogs thereof, are disclosed in the art for use in the manufacture of poly(aryl ether sulfone)s. Specifically, among the sulfur-containing solvents that may be suitable for the purposes of this invention are dimethylsulfoxide, dimethylsulfone, diphenylsulfone, diethylsulfoxide, diethylsulfone, diisopropylsulfone, tetrahydrothiophene-1,1-dioxide (commonly called tetramethylene sulfone or sulfolane) and tetrahydrothiophene-1-monoxide. Nitrogen-containing polar aprotic solvents, including dimethylacetamide, dimethylformamide and N-methyl-pyrrolidinone pyrrolidinone and the like have been disclosed in the art for use in these processes, and may also be found useful in the practice of the present invention.

The solvent that forms an azeotrope with water will necessarily be selected to be inert with respect to the monomer components and polar aprotic solvent. Those disclosed and described in the art as suitable for use in such polymerization processes include aromatic hydrocarbons such as benzene, toluene, xylene, ethylbenzene, chlorobenzene and the like.

The azeotrope-forming solvent and polar aprotic solvent are typically employed in a weight ratio of from about 1:10 to about 1:1, preferably from about 1:5 to about 1:1.

Generally, after an initial heat-up period, the temperature of the reaction mixture will be maintained in a range of from about 160°C to about 250°C, preferably from about 200°C to about 230°C, still more preferably from about 200°C to about 225°C for about 0.5 to 3 hours. Typically, if the reaction is conducted at atmospheric pressure, the boiling temperature of the solvent selected usually limits the temperature of the reaction.

The reaction may be conveniently carried out in an inert atmosphere, e.g., nitrogen, at atmospheric pressure, although higher or lower pressures may also be used.

It is essential that the reaction medium be maintained substantially anhydrous during the polycondensation. While amounts of water up to about one percent, preferably no more than 0.5 percent by weight, can be tolerated, and are somewhat beneficial when employed with fluorinated dihalobenzenoid compounds, amounts of water substantially greater than this are desirably avoided as the reaction of water with the halo compound leads to formation of phenolic species and low molecular weight products are obtained. Substantially anhydrous conditions may be conveniently maintained during the polymerization by removing water continuously from the reaction mass with the azeotrope-forming solvent as an azeotrope. In the preferred procedure, substantially all of the azeotrope-forming solvent, for example, chlorobenzene, will be removed by distillation as an azeotrope with the water formed in the reaction, leaving a solution comprising the poly(aryl ether sulfone) product dissolved in the polar aprotic solvent.

Sometimes, after the desired molecular weight has been attained, the polymer is endcapped to improve melt and oxidative stability. Generally, the endcapping is accomplished by adding a reactive aromatic halide or an aliphatic halide such as methyl chloride, benzyl chloride or the like to the polymerization mixture, converting any terminal hydroxyl groups into ether groups. In some instances, the polymer is intentionally left with excess hydroxyl groups to produce a reactive polymer. For the present invention it is preferred to use a reactive polymer.

The poly(aryl ether sulfone) is subsequently recovered by methods well known and widely employed in the art such as, for example, coagulation, solvent evaporation and the like. In the particular case of a reactive polymer, the recovery method must avoid reaching temperatures where the polymer will react. Frequently, in case a high excess of hydroxyl endgroups is desired, the polymer reaction is conducted with an excess of the bishydroxy monomer.

Poly(aryl ether sulfone), in particular polyphenylsulfone, has the advantage that it is especially durable with respect to heat and pressure. Moreover it isolates against heat as well as noise. In traditional sterilization apparatuses, a large amount of energy is consumed for heating the material in the metal or plastic sterilization chamber for each sterilization cycle (sometimes referred to also as "autoclaving cycle"). This heating of material is considerably reduced in the sterilization chamber according to the present invention.

A further problem with the heating of the sterilization chamber in a common sterilization apparatus is that the heat may be transported through the chamber walls to the housing, resulting in a risk of burning for the users when the sterilization apparatus housing is touched. Consequently, in a prior art sterilization apparatus, an extra isolation is a prerequisite. This problem is avoided in the sterilization chamber of the present invention.

Furthermore, the sterilization process, when vapour, water and so on is fed into the sterilization chamber in a known meander, tends to create noise within the chamber. By using the sterilization chamber of the present invention, this kind of noise does not leave the chamber due to the good sound isolating properties of poly(aryl ether sulfone), in particular polyphenylsulfone, resulting in an improved work environment for the users.

The sterilization chamber is preferably releasably mountable in a sterilization apparatus, whereby the sterilization device, whereby the chamber may be mounted in the sterilization device as an easily exchangeable component.

In a preferred embodiment of the invention, the sterilization chamber is manufactured in one continuous piece. In this embodiment, the mounting of the chamber in the sterilization apparatus as well as any exchange of it is simplified.

Further, it is possible to manufacture the sterilization chamber without joints or the like. The risk of bacterial build-up is thus reduced, and clean and hygienic surfaces within the sterilization chamber are easily achieved. Furthermore, the lack of joints in the chamber reduces wear.

Preferably, components such as inlets and outlets for steam, moisture and the like, are integrally formed with the sterilization chamber of the present invention. This reduces the number of required components and facilitates the mounting of the chamber in the sterilization apparatus. Also, the risk of mounting a component incorrectly is reduced.

In a preferred embodiment, the sterilization chamber is provided with (preferably integrally formed) tracks in which a sealing chamber door may be slidably mounted. Thereby, no hinges or the like are necessary, and associated stress on the chamber door material at the attachment points is avoided. Furthermore, the number of joints in the interface between the chamber and the sealing chamber door may be kept at a minimum.

The sterilization chamber of the present invention has preferably an inner volume of up to 50 liters, more preferably 5 to 40 liters, even more preferably 10 to 30 liters.

In the sterilization chamber of the present invention, an insert, preferably also made of a poly(aryl ether sulfone), more preferably made of polyphenylsulfone, for holding trays or the like comprising the matters to be disinfected may be arranged within the chamber in a known manner.

According to a preferred embodiment of the invention, the sterilization chamber is manufactured in one piece using injection molding of a polyphenylsulfone material. A reinforcing material such as glass fibres or the like may be added to the polyphenylsulfone material in order to create a strong chamber structure.

The aforementioned advantages and objects of the inventions are moreover achieved in accordance with the present invention by a sterilization apparatus, comprising the aforementioned chamber sterilization.

The sterilization apparatus of the present invention may be fed from the front, rear, side or from the top with articles to be sterilized. In addition, feed-through apparatuses are conceived. Moreover, the sterilization apparatus may be adapted to different kinds of uses, from small clinic apparatuses to large apparatuses as used in industry. The adjustment of the sterilization apparatus and the sterilization chamber used therein will depend for example on the demands regarding temperature and pressure stability.

Namely, the sterilization chamber may have different shapes, with a rounded or rectangular cross-section. It is however preferred with the present invention that the sterilization chamber has a rounded cross-section (cylindrical, ellipsoidal etc.) i.e., oval or essentially rectangular shapes are possible and may be chosen for specific applications. Corners and edges will however in general be avoided when the applied pressure is significant (i.e. around 2 bars).

The sterilization chamber may be produced in several pieces that are mounted thereafter together to the desired sterilization chamber. However, a single piece chamber is preferred in most applications. In order to form a sterilization chamber from several separate pieces, the pieces may be put together by plastic welding.

The sterilization chamber according to the present invention may comprise a filter, which is preferably made from polyphenylsulfone. Namely, a sterile file is required in order to prevent germs from penetrating into the holding space of the sterilization chamber following the sterilization operation. This filter has to be replaced from time to time and is thus preferably detachable from the sterilization chamber.

The invention is moreover directed to a process for the manufacture of a sterilization chamber of the present invention, comprising the step of injection moulding a polymer comprising at least 90 wt. % poly(aryl ether sulfone) into a mould which is adapted to provide said sterilization chamber.

The sterilization chamber and apparatus, and the process for the manufacture of the present sterilization chamber have a number of advantages. It was found that the sterilization chamber of the present invention retains the impact properties after steam sterilizations (for example 100 cycles) significantly better when compared to sterilization chambers that are made from other polymers, including polyetherimides and polyetherketones. In particular, the change in maximum load is significantly lower with the sterilization chamber of the present invention.

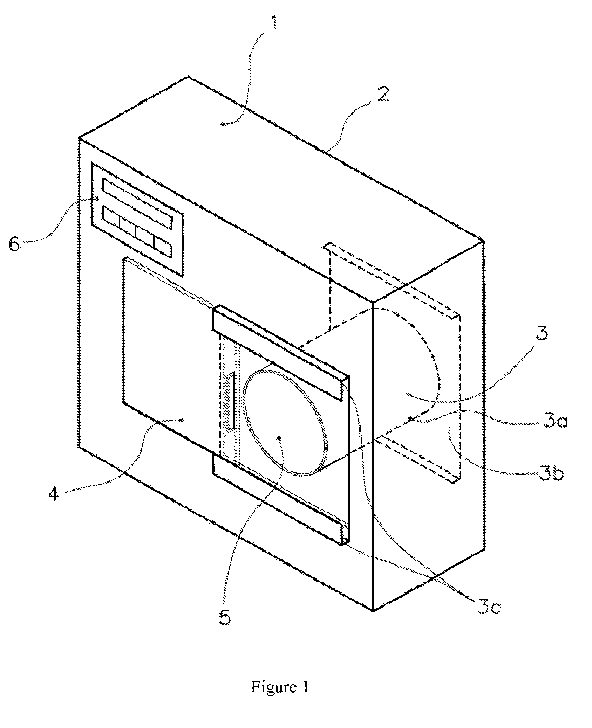

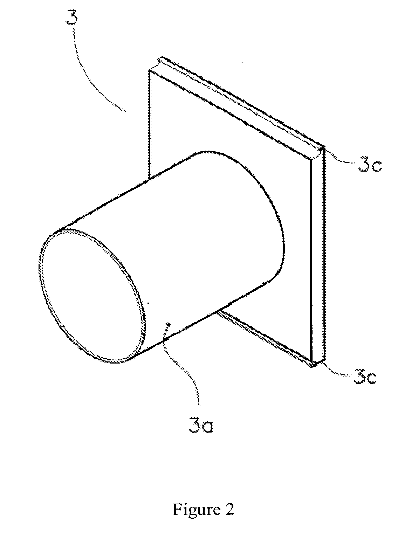

In the following, the invention is described in more detail with reference to Figs. 1 and 2 wherein non-limiting examples for the sterilization chamber and sterilization apparatus according to the present invention are shown.

Fig. 1 is a perspective view of a sterilization apparatus, comprising a sterilization chamber in accordance with the present invention. The sterilization apparatus 1 comprises a housing 2, in which a sterilization chamber 3 according to the invention is placed. The sterilization apparatus 1 also comprises customarily used pressurization means, vaporization means etc. (not shown here).

In the embodiment of the invention shown in Fig. 1, the sterilization chamber 3 comprises a cylindrical portion 3a having a back wall 3b, 3a and 3b together forming a container having a front opening 5 through which goods to be sterilized may be entered. The sterilization apparatus 1 moreover comprises a chamber door 4 that can be moved between a first position (opening 5 is closed) and a second position (opening 5 accessible). The chamber door 4 thus creates together with the inside of the sterilization chamber 3 (i.e. the cylindrical portion and the back wall) a sterilization enclosure. In this position, the door 4 is in sealing contact with the sterilization chamber 3. In the second position, the door 4 is removed from front opening 5, leaving the sterilization chamber 3 open for entering or removing goods. In the embodiment shown in Fig. 1, the door is slidably arranged between the first and the second position.

In accordance with the invention, the sterilization chamber 3 is manufactured from a poly(aryl ether sulfone). The chamber may be formed by injection moulding, casting or the like.

The sterilization chamber 3 is manufactured in one piece using injection molding of a poly(aryl ether sulfone), in particular a polyphenylsulfone material. A reinforcing material may be added to the poly(aryl ether sulfone)material in order to create a strong chamber structure. Said reinforcement material may be glass fibres or the like.

Moreover, sterilization chamber 3 shown in this embodiment is detachable from the sterilization apparatus 1.

The sterilization apparatus 1, in particular the sterilization chamber 3, from Fig. 1 is fed from the front side with articles to be sterilized. The sterilization chamber 3 has a cylindrical shape in order to resist especially good to high pressure.

As seen in Figure 1, sterilization chamber 3 comprises fastening portions, formed in integration with the remainder of the chamber. The fastening portions 3c are intended to be used for releasable mounting of the chamber in the sterilization device by means of fasteners (not shown herein). In the present case, the fastening portions are flat front and back surfaces 3c, provided with openings, through which a fastening means like a screw may be introduced and thereafter fastened in the housing 2. Other ways of mounting the sterilization chamber 3 in the sterilization apparatus are possible and may be selected according to need. Consequently, sterilization chamber 3 may easily be removed from sterilization apparatus 1, for example for replacement in case of wear or altered user needs.

In order to facilitate mounting, inlets for vapour, water and the like, and outlets for excess fluids, are integrally formed in the sterilization chamber of the embodiment shown in Fig. 1. Thereby the mounting and the exchange of the sterilization chamber are further facilitated. The placement and configuration of inlets and outlets in the chamber depend on the desires of the person skilled in the art.

As shown in Fig. 1, the chamber door 4 is slidably mounted in a pair of parallel tracks 3c, being formed in integration with sterilization chamber 3. Said tracks 3c encompass the door 4 on two opposite edges. Further, sealing means are provided on door 4 in order to provide for the creation of a sealed pressure chamber when door 4 is closed. By arranging door 4 as a slidable component, no hinges or the like need to be attached to the sterilization chamber that could result in high wear in the hinge fastening points. By utilizing this track approach, wear is avoided, resulting in a longer lifetime of the sterilization chamber.

In the shown embodiment of the invention, the sterilization chamber 3 is manufactured in one piece, i.e. without joints. This results in a clean and smooth inner surface of the chamber, resulting in a decreased risk for bacterial buildups. Furthermore, due to the used material's properties, condensation is less likely to appear on the poly(aryl ether sulfone) material than on other materials. Consequently, it is easier to obtain dry goods in the chamber, since there is less condensation of moisture, whereby the added energy may be used in the sterilization process instead of vaporizing condensate.

Fig. 2 shows an enlarged view of the sterilization chamber 3 of the present invention as used in the sterilization apparatus 1 shown in Fig. 1. 3a is the cylindrical portion of the sterilization chamber 3.

## Claims

1. A pressurizable sterilization chamber (3) made of a polymeric material, comprising at least one poly(biphenyl ether sulfone) (P1) and wherein said chamber comprises a filter for preventing germs from penetrating into the holding space of the sterilization chamber, **characterized in that** said filter is made of polyphenylsulfone.

2. The pressurizable sterilization chamber (3) according to claim 1, wherein the poly (biphenyl ether sulfone) (P1) is a polyphenylsulfone homopolymer.

3. The pressurizable sterilization chamber (3) according to any of the preceding claims, wherein the poly (biphenyl ether sulfone) (P1) has a weight average molecular weight in the range of from 20,000 to 100,000.

4. The pressurizable sterilization chamber (3) according to claim 3, wherein the poly (biphenyl ether sulfone) (P1) has a weight average molecular weight in the range of from 40,000 to 70,000.

5. The pressurizable sterilization chamber (3) according to any of the preceding claims, wherein the polymeric material comprises the poly (biphenyl ether sulfone) (P1) in a weight amount of at least 90 wt. %, based on the total weight of the polymeric material.

6. The pressurizable sterilization chamber (3) according to claim 5, wherein the polymeric material consists essentially of the poly (biphenyl ether sulfone) (P1).

7. The pressurizable sterilization chamber (3) according to any of the preceding claims, which consists essentially of the polymeric material.

8. The pressurizable sterilization chamber (3) according to any of the preceding claims, wherein said chamber is essentially manufactured in one continuous piece.

9. The pressurizable sterilization chamber (3) according to any of the preceding claims, wherein inlets and outlets for steam, moisture and the like, are integrally formed with said chamber (3).

10. The pressurizable sterilization chamber (3) according to any of the preceding claims, wherein said chamber (3) is provided with tracks, in which a sealing chamber door may be slidably mounted.

11. The pressurizable sterilization chamber (3) according to any of the preceding claims, **characterized in that** it has an inner volume of up to 50 liters.

12. The pressurizable sterilization chamber (3) according to any of the preceding claims, wherein said sterilization chamber is pressurizable at a pressure of over 2 bars.

13. A sterilization apparatus (1) comprising the pressurizable sterilization chamber (3) of any of the preceding claims.

14. A process for the manufacture of the pressurizable sterilization chamber (3) as defined in any of claims 1 to 12, which comprises the step of injection moulding the polymeric material into a mould which is adapted to provide said sterilization chamber (3).

## Patentansprüche

1. Druckbeaufschlagbare Sterilisationskammer (3) aus einem Polymermaterial, umfassend mindestens ein Poly(biphenylethersulfon) (P1) und wobei die Kammer einen Filter umfasst, um zu verhindern, dass Keime in den Halteraum der Sterilisationskammer eindringen, **dadurch gekennzeichnet, dass** der Filter aus Polyphenylsulfon hergestellt ist.

2. Druckbeaufschlagbare Sterilisationskammer (3) nach Anspruch 1, wobei das Poly(biphenylethersulfon) (P1) ein Polyphenylsulfon-Homopolymer ist.

3. Druckbeaufschlagbare Sterilisationskammer (3) nach einem der vorherigen Ansprüche, wobei das Poly(biphenylethersulfon) (P1) eine gewichtsmittlere Molmasse in dem Bereich von 20.000 bis 100.000 aufweist.

4. Druckbeaufschlagbare Sterilisationskammer (3) nach Anspruch 3, wobei das Poly(biphenylethersulfon) (P1) eine gewichtsmittlere Molmasse in dem Bereich von 40.000 bis 70.000 aufweist.

5. Druckbeaufschlagbare Sterilisationskammer (3) nach einem der vorherigen Ansprüche, wobei das Polymermaterial das Poly(biphenylethersulfon) (P1) in einer Gewichtsmenge von mindestens 90 Gew.-% umfasst, basierend auf dem Gesamtgewicht des Polymermaterials.

6. Druckbeaufschlagbare Sterilisationskammer (3) nach Anspruch 5, wobei das Polymermaterial im Wesentlichen aus dem Poly(biphenylethersulfon) (P1) besteht.

7. Druckbeaufschlagbare Sterilisationskammer (3) nach einem der vorherigen Ansprüche, die im Wesentlichen aus dem Polymermaterial besteht.

8. Druckbeaufschlagbare Sterilisationskammer (3) nach einem der vorherigen Ansprüche, wobei die Kammer im Wesentlichen als durchgehendes Teil hergestellt ist.

9. Druckbeaufschlagbare Sterilisationskammer (3) nach einem der vorherigen Ansprüche, wobei die Einlässe und Auslässe für Dampf, Feuchtigkeit und dergleichen einstückig mit der Kammer (3) ausgebildet sind.

10. Druckbeaufschlagbare Sterilisationskammer (3) nach einem der vorherigen Ansprüche, wobei die Kammer (3) mit Gleitführungen versehen ist, in denen eine Dichtungskammertür verschiebbar angebracht sein kann.

11. Druckbeaufschlagbare Sterilisationskammer (3) nach einem der vorherigen Ansprüche, **dadurch gekennzeichnet, dass** diese ein Innenvolumen von bis zu 50 Litern aufweist.

12. Druckbeaufschlagbare Sterilisationskammer (3) nach einem der vorherigen Ansprüche, wobei die Sterilisationskammer bei einem Druck von über 2 bar druckbeaufschlagbar ist.

13. Sterilisationsvorrichtung (1), umfassend die druckbeaufschlagbare Sterilisationskammer (3) nach einem der vorherigen Ansprüche.

14. Verfahren zum Herstellen der druckbeaufschlagbaren Sterilisationskammer (3) nach einem der Ansprüche 1 bis 12, wobei das Verfahren den Schritt des Spritzgießens des Polymermaterials in eine Gussform umfasst, die zum Bereitstellen der Sterilisationskammer (3) ausgelegt ist.

## Revendications

1. Chambre de stérilisation pouvant être mise sous pression (3) constituée d'un matériau polymère, comprenant au moins une poly(biphényléthersulfone) (P1) et ladite chambre comprenant un filtre pour empêcher des germes de pénétrer dans l'espace de rétention de la chambre de stérilisation, **caractérisée en ce que** ledit filtre est constitué de polyphénylsulfone.

2. Chambre de stérilisation pouvant être mise sous pression (3) selon la revendication 1, dans laquelle la poly(biphényléthersulfone) (P1) est un homopolymère de polyphénylsulfone.

3. Chambre de stérilisation pouvant être mise sous pression (3) selon l'une quelconque des revendications précédentes, dans laquelle la poly(biphényléthersulfone) (P1) a une masse moléculaire moyenne en poids de 20 000 à 100 000.

4. Chambre de stérilisation pouvant être mise sous pression (3) selon la revendication 3, dans laquelle la poly(biphényléthersulfone) (P1) a une masse moléculaire moyenne en poids de 40 000 à 70 000.

5. Chambre de stérilisation pouvant être mise sous pression (3) selon l'une quelconque des revendications précédentes, dans laquelle le matériau polymère comprend la poly(biphényléthersulfone) (P1) dans une quantité pondérale d'au moins 90 % en poids, relativement au poids total du matériau polymère.

6. Chambre de stérilisation pouvant être mise sous pression (3) selon la revendication 5, dans laquelle le matériau polymère se compose essentiellement de la poly(biphényléthersulfone) (P1).

7. Chambre de stérilisation pouvant être mise sous pression (3) selon l'une quelconque des revendications précédentes, constituée essentiellement du matériau polymère.

8. Chambre de stérilisation pouvant être mise sous pression (3) selon l'une quelconque des revendications précédentes, ladite chambre étant fabriquée essentiellement en une seule pièce.

9. Chambre de stérilisation pouvant être mise sous pression (3) selon l'une quelconque des revendications précédentes, les entrées et sorties de vapeur, d'humidité et de matières similaires faisant partie intégrante de ladite chambre (3).

10. Chambre de stérilisation pouvant être mise sous pression (3) selon l'une quelconque des revendications précédentes, ladite chambre (3) étant pourvue de glissières, dans lesquelles une porte assurant l'étanchéité de la chambre peut être montée de manière coulissante.

11. Chambre de stérilisation pouvant être mise sous pression (3) selon l'une quelconque des revendications précédentes, **caractérisée en ce qu'**elle a un volume intérieur de jusqu'à 50 litres.

12. Chambre de stérilisation pouvant être mise sous pression (3) selon l'une quelconque des revendications précédentes, ladite chambre de stérilisation pouvant être mise sous pression à une pression de plus de 2 bars.

13. Appareil de stérilisation (1) comprenant la chambre de stérilisation pouvant être mise sous pression (3) selon l'une quelconque des revendications précédentes.

14. Procédé de fabrication de la chambre de stérilisation pouvant être mise sous pression (3) selon l'une quelconque des revendications 1 à 12, comprenant l'étape de moulage par injection du matériau polymère dans un moule qui est conçu pour produire ladite chambre de stérilisation (3).
